# EUROPÄISCHE PATENTSCHRIFT

(11) **EP 3 219 782 B1**
(45) Veröffentlichungstag und Bekanntmachung des Hinweises auf die Patenterteilung: **05.09.2018**
(21) Anmeldenummer: 16206829.0
(22) Anmeldetag: 23.12.2016
(51) Int. Cl.: C12M 1/00, C12M 1/22, C12M 3/00

(54) **ZUFÜHRVORRICHTUNG**
SUPPLY DEVICE
DISPOSITIF D'AMENÉE

(30) Priorität: 01.03.2016 DE 102016103639
(43) Veröffentlichungstag der Anmeldung: 20.09.2017
(73) Patentinhaber: Andreas Hettich GmbH & Co. KG, 78532 Tuttlingen (DE)
(72) Erfinder: Eberle, Klaus-Günter, 78532 Tuttlingen (DE)
(74) Vertreter: Puschmann Borchert Bardehle Patentanwälte Partnerschaft mbB

(56) Entgegenhaltungen:
- EP-A1- 1 018 544
- EP-A2- 2 482 079
- IUL Instruments: "Plate Handler", , 28. Februar 2016 (2016-02-28), XP002772493, Gefunden im Internet: URL:http://www.iul-inst.com/images/6094R01 .pdf [gefunden am 2017-07-25]

## Beschreibung

Die Erfindung betrifft ein Zuführsystem für Laborgefäße gemäß der im Oberbegriff des Patentanspruches 1 angegebenen Art.

Aus dem Stand der Technik sind verschiedene Ansätze bekannt, Systemen zur Verarbeitung und Analyse von Mikroorganismen und Zellkulturen Laborgefäße, gewöhnlich Petrischalen, zuzuführen und dabei manuelle Tätigkeiten eines Benutzers auf ein Minimum zu reduzieren. Es ist üblich, Petrischalen stapelweise in einen Ladebereich einzubringen und von dort einzeln zum nächsten Schritt, etwa in eine Analyse- oder Präparationseinheit, weiter zu fördern. Ferner wird versucht, mehrere Stapel auf einmal in das System einzubringen, um die Abstände zwischen den Beladevorgängen zu vergrößern und dem Benutzer dadurch Zeit für andere Tätigkeiten zu verschaffen.

Beispielsweise schlägt die IUL, S.A. einen Probenwechsler (Plate Handler) mit einem integrierten Magazin vor, in welches ein mit Petrischalen befüllter Petrischalenträger eingesetzt wird. Der Träger weist vier Aufnahmen für übereinander gestapelte Petrischalen auf und ist im eingesetzten Zustand nach Art eines Karussells um die eigene Achse drehbar. Für die Rotationsbewegung des Trägers ist ein herkömmlicher Antriebsmotor vorgesehen. In einer Entladezone wird ein Stapel Petrischalen aus einer Aufnahme entnommen und horizontal in Richtung der Analyseeinheit bewegt. In dieser Position ist ein Lift vorgesehen, mittels dem die Petrischalen einzeln in die Analyseeinheit geladen und nach der Analyse wieder aus der Analyseeinheit entnommen werden. Nach der Analyse sämtlicher im Stapel befindlichen Petrischalen wird der Stapel wieder zum Träger zurück bewegt und in die ursprüngliche Aufnahme eingebracht. Anschließend wird das Magazin durch eine Fördereinheit weitergedreht, so dass Petrischalen aus der nächsten befüllten Aufnahme entnommen werden können.

Diese Lösung ermöglicht es, bis zu vier Stapel Petrischalen in das Magazin zu laden und diese Petrischalen ohne weiteres Zutun des Benutzers der Analyseeinheit zuzuführen. Jedoch verbleibt der Träger im Magazin und steht während des gesamten Analysevorgangs nicht zum Beladen und Transportieren weiterer Petrischalen zur Verfügung. Zudem besteht beim Entnehmen jeweils eines gesamten Stapels Petrischalen aus einer Aufnahme und beim anschließenden Wiedereinsetzen die Gefahr, dass der Stapel instabil wird und die Petrischalen verrutschen oder gar herunterfallen. Ferner ist der Entladebereich schwer zugänglich, und es muss unter Umständen erst der Träger entfernt werden, um eine Störung zu beheben.

Dokument WO2016170161 offenbart einen Petrischalenträger zum Aufnehmen und Lagern von Petrischalen, wobei der Träger eine Mehrzahl von Aufnahmen aufweist, in die Petrischalen einbringbar sind und in denen die Laborgefäße lagerbar sind. Dokument DE102010044125 offenbart eine Petrischalen-Befüll- und Zuführeinrichtung. Ein Karussell mit Schalenstapeln kann vorab und räumlich von der Befüllvorrichtung getrennt und mit leeren Petrischalen bestückt werden und dann darauf nachfolgend einzeln befüllt werden. Sind die einzelnen Petrischalen befüllt, kann ein optischer oder akustischer Hinweis erfolgen. Dokument EP2482079 offenbart eine Vorrichtung zur Lagerung und Handhabung von Petrischalen. Die Transfervorrichtung weist im Bereich des Greifers Sensoren auf, die zur Detektion des Vorhandenseins bzw. Nichtvorhandenseins der Petrischale Rückschlüsse liefert.

In der Druckschrift WO2016170161 ist ein Träger zum Aufnehmen und Lagern von Laborgefäßen offenbart, durch den bereits ein Ansatz für die Lösung der oben erläuterten Problematik aufgezeigt wird.

Der Träger weist eine Mehrzahl von Aufnahmen auf, in die Laborgefäße einbringbar sind und in denen die Laborgefäße lagerbar sind. An einer Oberseite des Trägers ist jeweils eine Ladeöffnung an jeder Aufnahme vorgesehen, und an einer Unterseite jeweils eine durch einen Verschließmechanismus verschließbare Entladeöffnung. Zum Beladen des Trägers mit Laborgefäßen sowie zum Transport ist die Entladeöffnung verschlossen. Nach dem Einsetzen des Trägers in ein Magazin einer Zuführvorrichtung für Laborgefäße wird der Verschließmechanismus geöffnet und der Träger wird aus dem Magazin entnommen, wobei die Laborgefäße im Magazin verbleiben. So gelangen die Stapel von Laborgefäßen in den Aufnahmen des Trägers sicher in das Magazin. Während der Analyse steht der Träger jedoch zur Lagerung und zum Transport anderer Laborgefäße zur Verfügung. Zudem sind die Laborgefäße im Magazin ohne den Träger besser zugänglich, so dass eine Störung einfacher behoben werden kann.

Allerdings hat es sich in der Praxis mitunter als schwierig erwiesen, die Position bestimmter Laborgefäße nach dem Einbringen in das Magazin zu jeder Zeit eindeutig anzugeben. Insbesondere bei Fehlfunktionen von Sensoren zur Lagebestimmung von Laborgefäßen kann es im Falle einer mechanischen Störung dazu kommen, dass es unklar ist, welche Laborgefäße bereits in Abwesenheit des Bedieners verarbeitet wurden.

Aufgabe der Erfindung ist es, unter Vermeidung der genannten Nachteile ein Zuführsystem derart weiterzubilden, dass die Bestimmung der exakten Position Laborgefäße innerhalb der Zuführvorrichtung zu jeder Zeit, auch bei Störungen, möglich ist.

Diese Aufgabe wird durch die kennzeichnenden Merkmale des Patentanspruches 1 in Verbindung mit seinen Oberbegriffsmerkmalen gelöst.

Die Unteransprüche bilden vorteilhafte Weiterbildungen der Erfindung.

Der Erfindung liegt die Erkenntnis zugrunde, dass es die Bestimmung der Position von Laborgefäßen, insbesondere von Stapeln von Laborgefäßen, innerhalb der Zuführvorrichtung vereinfacht, wenn ein eindeutig festgelegtes Schema beim Befüllen der Zuführvorrichtung durch den Träger vorgegeben ist.

Der erfindungsgemäßen Ausgestaltung entsprechend weist der Träger zumindest zwei Aufnahmen auf, und der Formschluss zwischen Träger und Aufnahmeeinheit im Ladebereich der Zuführvorrichtung ermöglicht nur eine einzige vorbestimmte Ausrichtung des Trägers im Ladebereich. So ist beim Laden der Laborgefäße aus dem Träger in den Ladebereich eine eindeutige Zuordnung eines oder mehrerer Stapel von Laborgefäßen zu einer Aufnahmeeinheit möglich. Dies erleichtert die gleichzeitige Handhabung unterschiedlich befüllter Laborgefäße und verringert die Wahrscheinlichkeit von Verwechslungen. Verwechslungen von Proben können fatale Folgen haben, z.B. falsche Diagnosen bei Patientenproben usw. Eine verringerte Wahrscheinlichkeit für Verwechslungen erhöht die Patientensicherheit und vermindert das Risiko von falschen Diagnosen. Zudem kann dadurch beträchtlich Zeit eingespart werden.

In einer weiteren vorteilhaften Ausgestaltung kann die Aufnahmeeinheit so ausgebildet sein, dass sie, insbesondere bei einer Störung, durch die Endlosfördereinheit in den Ladebereich zurück verfahrbar ist. Bei einer Störung insbesondere mechanischer Art ist es häufig günstiger, die in die Störung involvierte Aufnahmeeinheit entgegen der Richtung, aus der sie transportiert wurde, aus der Störungszone heraus zu verfahren. Dadurch wird das Beheben der Störung vereinfacht, und der Betrieb der Zuführvorrichtung wird sicherer.

Die Endlosfördereinheit hat insbesondere eine Grundposition, die vorzugsweise durch einen optoelektronischen Sensor überprüft werden kann. Durch diese Referenzierung ist eine eindeutige Nummerierung und somit die Zuordnung der Stapel möglich. Im Falle eines elektrischen Versagens, beispielsweise Stromausfall, etc., fährt das Endlosförderband in die Grundposition. Der Stapel kann dann weiterhin eindeutig zugeordnet werden.

Günstig ist es, wenn eine Steuerelektronik vorgesehen ist, welche die Lage der Aufnahmeeinheit in dem Ladebereich bei der Beladung erfasst und speichert, so dass bedarfsweise die Aufnahmeeinheit in ihre ursprüngliche Lage im Ladebereich zurückverfahren kann und somit einer vorbestimmten Aufnahme im in den Ladebereich eingebrachten Träger zugeordnet bleibt. Im Fall einer Störung können in der Zuführvorrichtung verbliebene Laborgefäße dann durch einen Träger wieder entnommen und in derselben Anordnung in eine andere Zuführvorrichtung eingebracht werden. Dies erhöht den Automatisierungsgrad des Zuführsystems und erspart Zeit.

Bei einer vorteilhaften Weiterbildung der Erfindung ist ein Sensor, insbesondere ein Tastsensor, im Ladebereich der Zuführvorrichtung vorgesehen, der erfasst, ob ein Träger in dem Ladebereich angeordnet ist. Der Sensor wirkt vorzugsweise mit der Steuerelektronik so zusammen, dass ein Fördern der Endlosfördereinheit blockiert wird, wenn ein Träger im Ladebereich angeordnet ist. So wird eine mechanische Beschädigung der Zuführvorrichtung verhindert, wodurch ihre Lebensdauer verlängert wird. Dieser Sensor kann ein Mikroschalter sein, der nicht nur ein Fördern mit eingesetztem Träger unterbindet, sondern auch den Öffnungs- und Schließvorgang für den Träger nur zulässt, wenn dieser eingesetzt ist. Vorzugsweise kann eine statische, mechanische Positionsabfrage aus Kunststoff vorgesehen sein, d.h. der Träger besitzt eine Ausnehmung und nur wenn dieser korrekt eingeführt wird, lässt sich dieser vollständig einsetzen, den Mikroschalter aktivieren und demnach öffnen bzw. schließen. Der Vorteil des "richtigen" Einsetzens, auch wenn der Träger symmetrisch ausgebildet ist, liegt wiederum in der eindeutigen Lagebestimmung der verschiedenen Stapel. Die Aufnahmen des Trägers für die Stapel tragen eindeutige Bezeichnungen, was die Zuordnung erleichtert. Alternativ ist der Träger asymmetrisch ausgebildet.

Gemäß einem Aspekt der Erfindung ist im Ladebereich ein Entriegelungsmechanismus für den Träger vorgesehen, der bedarfsweise einen korrekt in den Ladebereich eingebrachten Träger öffnet, so dass Laborgefäße in eine Aufnahmeeinheit überführt werden können, oder schließt, so dass in einer Aufnahmeeinheit befindliche Laborgefäße wieder entnommen werden können. Dadurch sind der Träger und die Zuführvorrichtung besser in das Zuführsystem integriert, und in Verbindung mit dem zuvor beschriebenen Tastsensor kann ein höherer Automatisierungsgrad erreicht werden.

Es ist zweckmäßig, dass im Ladebereich Sensoren vorhanden sind, welche erfassen, ob in der Aufnahmeeinheit zumindest ein Laborgefäß vorhanden ist, wobei die Sensoren vorzugsweise durch optoelektronische Sensoren wie Lichtschranken gebildet werden. Durch die optoelektronischen Sensoren ist eine berührungslose Messung möglich. Es gibt eine gute elektromagnetische Verträglichkeit. Unbefüllte Aufnahmeeinheiten können dadurch sofort von der Endlosfördereinheit weitertransportiert werden, und es wird sichergestellt, dass im Entnahmebereich nur auf befüllte Aufnahmeeinheiten zugegriffen wird. Dies kann beträchtlich viel Zeit einsparen.

Vorzugsweise begrenzen die Vorsprünge für den Formschluss des Trägers zugleich auch die in die Aufnahmeeinheit eingebrachten Laborgefäße seitlich. Dies vereinfacht die Konstruktion und senkt die Kosten des Zuführsystems.

Bei einer bevorzugten Ausführungsform ist einer Aufnahme des Trägers lediglich eine Aufnahmeeinheit zugeordnet. So ist eine Zuordnung eines Stapels von Laborgefäßen innerhalb der Zuführvorrichtung noch einfacher, und das Risiko einer falschen Referenzierung sinkt.

Bei einer vorteilhaften Weiterbildung der Erfindung weist der Träger eine bestimmte Zahl von Aufnahmen für die Laborgefäße auf, und der Ladebereich der Zuführvorrichtung ist durch eine Zahl von Aufnahmeeinheiten gebildet, welche der Zahl von Aufnahmen des Trägers entspricht. Bei eingebrachtem Träger können somit alle Aufnahmeeinheiten im Ladebereich beladen oder entladen werden. Dadurch wird, insbesondere in Verbindung mit dem oben beschriebenen Formschluss zwischen Träger und Zuführvorrichtung, eine falsche Referenzierung der Stapel von Laborgefäßen nach dem Einsetzen des Trägers nahezu ausgeschlossen. Dies verbessert die Zuverlässigkeit des Zuführsystems deutlich.

Gemäß einem weiteren Aspekt der Erfindung besteht die Endlosfördereinheit aus geradlinigen und kurvenförmigen Abschnitten. Insbesondere wenn sich der Entladebereich im kurvenförmigen Abschnitt der Endlosfördereinheit befindet, wird rein geometrisch schon ein Laden während des Entladens verhindert, was wiederum die Verwechslungsgefahr von Stapeln und Proben reduziert.

Vorzugsweise ist ein ganzer Stapel von Laborgefäßen als Stapeleinheit von oben her mittels des Trägers in eine Aufnahmeeinheit einbringbar, insbesondere vier Stapeleinheiten auf einmal in vier Aufnahmeeinheiten. In einem Beladevorgang kann folglich eine größere Zahl von Laborgefäßen in die Zuführvorrichtung eingebracht werden, wodurch die Bedienung des Zuführsystems erleichtert wird.

Um das Handling der Laborgefäße zu vereinfachen, ist es von Vorteil, wenn jeder Stapel von Laborgefäßen jeweils den gleichen Typen von Kulturen aufweist. So können die Laborgefäße besser für die nachfolgende Analyse o. Ä. in Gruppen zusammengefasst werden, und die Zuordnung einzelner Typen von Kulturen wird erleichtert.

Wenn jedes Laborgefäß und jeder Stapel gekennzeichnet ist, so dass jedes Laborgefäß und jeder Stapel einer Position im Ladebereich und einer Position im Träger zuordenbar ist, sinkt das Risiko einer fehlerhaften Zuordnung erheblich. Dies hat eine Verbesserung der Benutzerfreundlichkeit und der Zuverlässigkeit des Zuführsystems zur Folge. Beispielsweise können Barcodes in Verbindung mit entsprechenden Lesegeräten verwendet werden.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine Perspektivansicht des Zuführsystems von unten mit befülltem Träger in einer Position vor dem Einbringen in die Zuführvorrichtung;
- Fig. 2: eine Perspektivansicht der Zuführvorrichtung mit vier in einen Ladebereich der Zuführvorrichtung eingebrachten Petrischalenstapeln;
- Fig. 3: eine Perspektivansicht der Zuführvorrichtung mit einem in einen Entladebereich der Zuführvorrichtung eingebrachten Petrischalenstapel;
- Fig. 4: eine Perspektivansicht der Zuführvorrichtung mit einer in einem Übergabebereich lagernden Petrischale;
- Fig. 5: eine Ausführungsform der Erfindung mit zwei parallel zueinander angeordneten Zuführvorrichtungen.

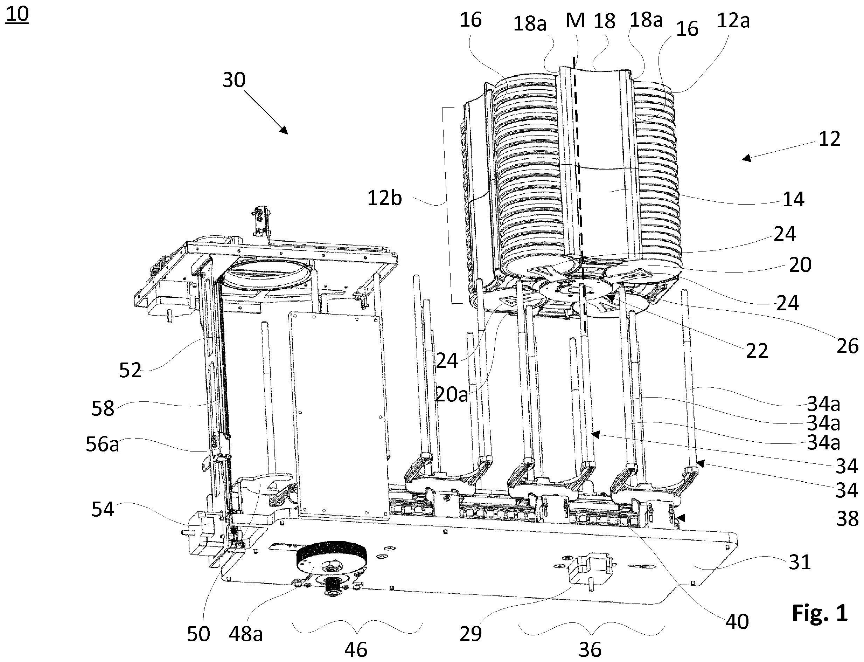

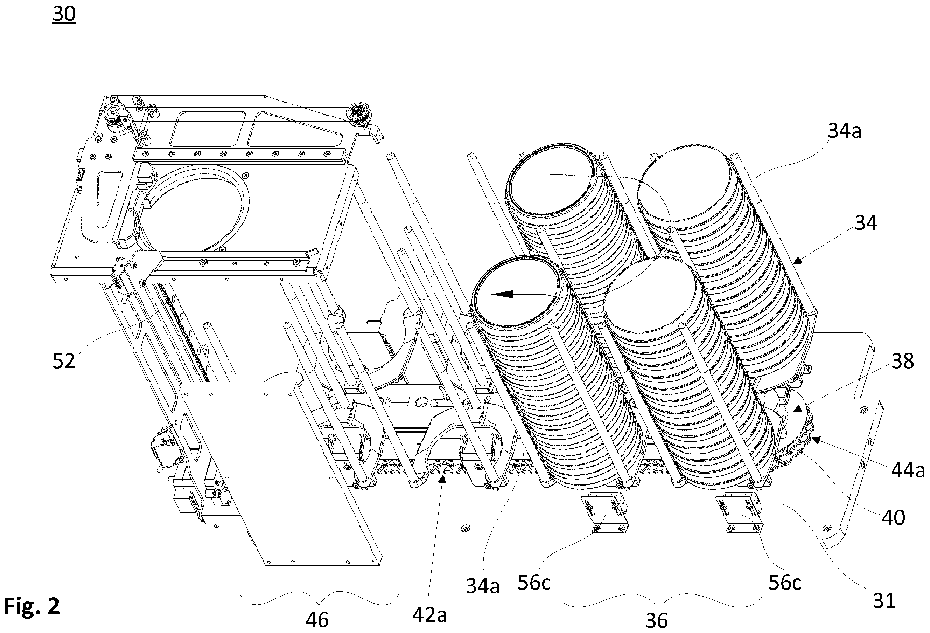

Fig. 1 zeigt eine Perspektivansicht des Zuführsystems 10 von unten umfassend einen Träger und eine Zuführvorrichtung 30. Der Träger 12 ist mit übereinander zu einem Stapel 12b angeordneten Petrischalen 12a befüllt und in einer Position vor dem Einsetzen in die Zuführvorrichtung 30 gezeigt. In den Figuren 2 bis 4 ist die Zuführvorrichtung 30 in unterschiedlichen Beladungszuständen dargestellt.

In ein Gehäuse 14 des Trägers 12 sind vier sternförmig zueinander angeordnete Aufnahmen 16 für übereinander gestapelte Petrischalen 12a eingebracht. Die Aufnahmen 16 sind nach außen hin geöffnet, allerdings nur soweit, dass zwar ein Eingriff von außen her möglich ist, eine Entnahme der Petrischalen 12a jedoch nur durch vertikales Verschieben der Schalen durch eine obere Ladeöffnung 18a der Aufnahme 16 möglich ist und keine Schalen aus dem Träger 12 fallen können. Der Anwender kann bequem seitlich auf die Petrischalen 12a zugreifen, diese aber nur von oben herausnehmen und die Petrischalen 12a jedoch ohne Gefahr des Herausfallens und einer möglichen daraus folgenden Kontamination sicher transportieren. Das Problem einer herausfallenden und sich öffnenden Petrischale 12a wäre gravierend. Es könnten gefährliche Erreger freigesetzt werden und die Umgebung kontaminieren. Darüber hinaus gibt es sehr wertvolle Proben, z.B. Knochenmarksproben von Kindern, die mit größter Vorsicht behandelt werden müssen.

Die Ladeachsen der Aufnahmen 16 verlaufen parallel zueinander und zu einer Mittelachse M des Trägers 12. An einer Oberseite 18 des Trägers 12 ist im Gehäuse 14 jeweils die Ladeöffnung 18a ausgebildet, über die die jeweilige Aufnahme 16 entlang der Ladeachse mit Petrischalen 12a beladen wird. Entsprechend ist an einer Unterseite 20 jeweils eine Entladeöffnung 20a im Gehäuse 14 ausgebildet, über die die Petrischalen 12a entladen werden.

Zum Transport und zum Schutz vor einer versehentlichen Entladung der Petrischalen 12a ist an der Unterseite 20 des Trägers 12 ein Verschließmechanismus 22 vorgesehen, der die Entladeöffnungen 20a der Aufnahmen 16 verschließt. Der Verschließmechanismus 22 umfasst vier Schieber 24, die im verschlossenen Zustand des Verschließmechanismus 22 die in die Aufnahmen 16 eingebrachten Petrischalen 12a überdecken und dadurch entlang der Ladeachse in Richtung zur Unterseite 20 festlegen.

Die Schieber 24 sind paarweise und im geschlossenen Zustand jeweils um 90° versetzt zueinander konzentrisch zur Mittelachse M des Trägers 12 angeordnet. Die beiden Paare von Schiebern 24 sind über ein der besseren Übersichtlichkeit halber nicht dargestelltes Getriebe gekoppelt, insbesondere in der Art eines Planetengetriebes, und mit einem Antrieb verbindbar. Für genauere Erläuterungen des Verschließmechanismus 22 wird auf die Anmeldung DE 10 2015 207 617.2 verwiesen. Insoweit wird auf die Offenbarung dieser Druckschrift Bezug genommen.

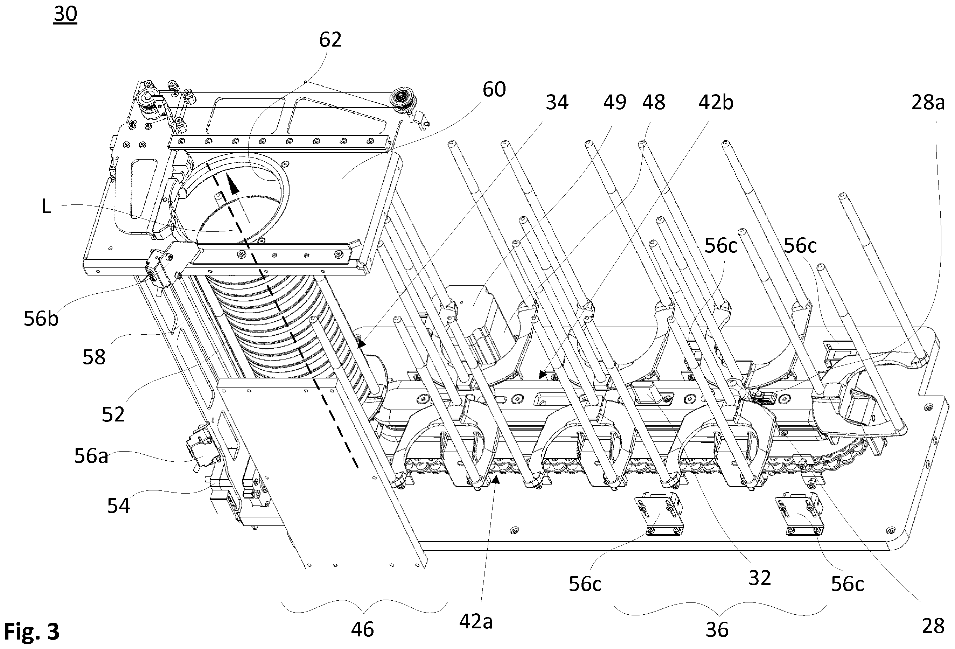

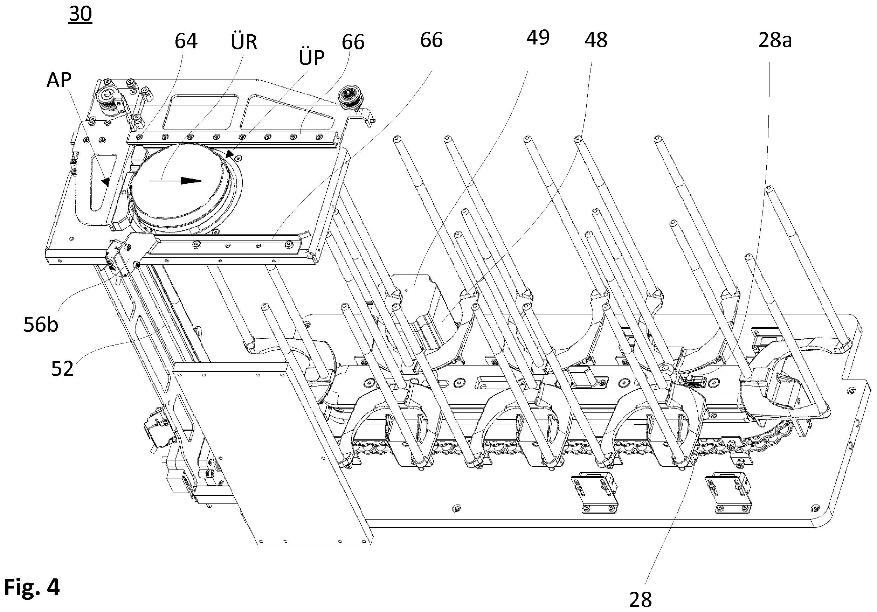

An der Unterseite 20 ist ferner zwischen zwei Aufnahmen 16 eine Ausnehmung 26 in das Gehäuse 14 eingebracht. In der Zuführvorrichtung 30 ist ein in Fig. 3 dargestellter mit der Ausnehmung 26 korrespondierender Zapfen 28 vorgesehen. Beim korrekten Einsetzen des Trägers 12 in die Zuführvorrichtung 30 greift der Zapfen 28 formschlüssig in die Ausnehmung 26 ein. Ein vollständiges Einsetzen des Trägers 12 kann nur bei Formschluss zwischen Ausnehmung 26 und Zapfen 28 erfolgen. Zugleich wird, wenn der Zapfen 28 in die Ausnehmung 26 eingreift, das oben erwähnte Getriebe aktiviert, das eine rotatorische Bewegung der paarweise angeordneten Schieber 24 bewirkt, so dass der Verschließmechanismus 22 geöffnet oder geschlossen wird. Die Aktivierung erfolgt durch einen neben dem Zapfen 28 angeordneten Mikroschalter 28a, siehe Figur 3 und 4, wobei ein Signal an eine Steuereinheit 49 gesendet wird, welche die Bewegung des Zapfen 28 bewirkt, wodurch der Träger 12 geöffnet oder geschlossen werden kann. In dieser Ausführung wird das Schließen und Öffnen über ein externes User-Interface vom Bediener initiiert. Dies ist aber nur bei korrekt eingesetztem Träger 12 überhaupt möglich. Dafür ist der Zapfen 28 mit einem Antriebsmotor 29, der unter der Zuführvorrichtung 30 angeordnet ist, drehfest verbunden. Die Steuereinheit 49, die im Zusammenhang mit den Figuren 3 und 4 noch weiter erläutert wird, steuert den Antriebsmotor 29 an, der eine Drehbewegung des Zapfens 28 verursacht, die wiederum den Verschließmechanismus 22 aktiviert oder deaktiviert.
Wird ein beladener Träger 12 mit geschlossenem Verschließmechanismus 22 in die Zuführvorrichtung 30 eingesetzt, bewirkt eine Drehbewegung des Zapfens 28 eine Öffnung des Verschließmechanismus 22. Ebenso ist es beispielsweise im Falle einer Störung möglich, einen unbeladenen Träger 12 mit geöffnetem Verschließmechanismus 22 in die mit Petrischalen 12a beladene Zuführvorrichtung 30 einzusetzen, den Verschließmechanismus 22 über eine Drehbewegung des Zapfens 28 zu schließen und den Träger 12 mit den Petrischalen 12a zu entnehmen.
Wird der Träger 12 in einer anderen als der vorgesehenen, baulich durch Ausnehmung 26 und Zapfen 28 und über eine Positionserfassung 32 festgelegten, Ausrichtung eingebracht, lässt sich der Träger 12 nicht vollständig einsetzen, und eine Öffnung des Verschließmechanismus 22 wird verhindert. Fig. 2 zeigt eine Perspektivansicht der Zuführvorrichtung 30 mit vier in einen Ladebereich 36 der Zuführvorrichtung 30 eingebrachten Stapeln 12b von Petrischalen 12a. Die Stapel 12b von Petrischalen 12a lagern in Aufnahmeständern 34, die jeweils drei parallel zueinander angeordnete, vertikal ausgerichtete Stangen 34a aufweisen, welche bezogen auf den Umfang der Petrischalen 12a gleichmäßig voneinander beabstandet sind.

Die Aufnahmeständer 34 sind auf einer Endlosfördereinheit 38 angeordnet, die einen in dieser Figur nicht dargestellten Antrieb und eine umlaufende Förderkette 40 aufweist. Die Förderkette 40 verläuft auf einer Bahn, die zwei zueinander und zur Längsachse der Zuführvorrichtung 30 parallel verlaufende geradlinige Abschnitte 42a, 42b aufweist, wobei der Abschnitt 42b besser in Fig. 3 erkennbar ist, und zwei kurvenförmige Abschnitte 44a, 44b. Dabei liegt der kurvenförmige Abschnitt 44a neben dem Ladebereich 36 und der kurvenförmige Abschnitt 44b neben dem an den Ladebereich 36 angrenzenden Entladebereich 46, wobei der kurvenförmige Abschnitt 44b in der für die Figuren gewählten Perspektive verdeckt ist. Die Endlosfördereinheit 38 ist auf einer Bodenplatte 31 der Zuführvorrichtung 30 festgelegt. Zudem ist ein Home-Switch zum Auffinden der Grundposition für die Endlosfördereinheit 38 vorgesehen. Hierfür ist auf der Förderkette 40 ein Reflektor in Form eines kleinen Plättchens befestigt, das mit einer Gabellichtschranke zusammenwirkt. Bei Durchfahren des Reflektors durch die Gabellichtschranke befindet sich die Endlosfördereinheit 38 in der Grundposition. Entsprechend ist der Reflektor und die Gabellichtschranke angeordnet.

Ein in Fig. 1 unterhalb der Zuführvorrichtung 30 teilweise ersichtlicher Antriebsmotor 48 ist als Antrieb der Endlosfördereinheit 38 vorgesehen und mit der Steuereinheit 49 in herkömmlicher Weise verbunden. Der Antriebsmotor 48 sitzt auf der Bodenplatte, siehe Fig. 3, an der Unterseite sieht man das Übersetzungsgetriebe 48a mittels Zahnräder und Zahnriemen. Mittels des Antriebsmotors 48 kann die Förderkette 40 samt den darauf aufgebrachten Aufnahmeständern 34 sowohl im Uhrzeigersinn als auch gegen den Uhrzeigersinn bewegt werden. Im Ladebereich 36 der Zuführvorrichtung 30 ist die Positionserfassung 32 auf der Endlosfördereinheit 38 angebracht.. Mit dem Einsetzen eines Trägers 12 im Ladebereich 36 wird der Mikroschalter 28a aktiviert und sendet ein Signal an die Steuereinheit 49, die daraufhin den Antriebsmotor 48 blockiert, so dass eine Bewegung der Endlosfördereinheit 38 mit eingesetztem Träger 12 verhindert wird. Der Zapfen 28 sowie die Positionserfassung verhindert ein vollständiges Einsetzen des Trägers in einer anderen Richtung als der vorgegebenen. Der Mikroschalter 28a, siehe Fig. 3, verhindert sowohl eine Bewegung der Fördereinheit und lässt auch nur ein Laden bzw. Entladen des Trägers nur im aktiven Zustand zu.

Nach der Entnahme des Trägers 12, bei der die Petrischalen 12a in der Zuführvorrichtung 30 verbleiben, entfällt das Signal des Mikroschalters 28a und die Steuereinheit 49 gibt den Antriebsmotor 48frei. Zudem sind im Ladebereich 36 auf der Bodenplatte 31 mit der Steuereinheit 49 verbundene fotoelektrische Sensoren 56c vorgesehen, die erfassen, ob in den im Ladebereich 36 befindlichen Aufnahmeständern 34 jeweils zumindest eine Petrischale 12a vorhanden ist.

Mit Stapeln 12b von Petrischalen 12a befüllte Aufnahmeständer 34 werden im Uhrzeigersinn aus dem Ladebereich 36 in den Entladebereich 46 gefördert, so dass vier weitere unbeladene Aufnahmeständer 34 zur Aufnahme von Stapeln 12b von Petrischalen 12a aus einem weiteren Träger 12 zur Verfügung stehen. So kann die Zuführvorrichtung 30 gleichzeitig mit bis zu acht Stapeln 12b von Petrischalen 12a beladen sein. Die sowohl im Uhrzeigersinn als auch gegen den Uhrzeigersinn bewegbare Endlosfördereinheit 38 gewährleistet, dass die Stapeln 12b von Petrischalen 12a in der gewünschten Reihenfolge abgearbeitet werden können. Zudem ist es im Fall einer Störung je nach aktueller Förderposition möglich, die Aufnahmeständer 34 gegen den Uhrzeigersinn schneller wieder in die Position zu bringen, die sie beim Einsetzen des Trägers 12 innehatten, und die Stapeln 12b von Petrischalen 12a in ihrer ursprünglichen Anordnung relativ zueinander wieder zu entnehmen.

In Fig. 3 ist der besseren Übersichtlichkeit nur ein in den Entladebereich 46 der Zuführvorrichtung 30 eingebrachter Stapeln 12b von Petrischalen 12a dargestellt. Über dem Entladebereich 46 ist parallel zur Bodenplatte 31 der Zuführvorrichtung 30 eine Übergabeplatte 60 angeordnet, die über eine lineare Verbindungsschiene 58 zur Bodenplatte 31 beabstandet ist. Die Übergabeplatte 60 dient dazu, wie im Folgenden beschrieben, Petrischalen 12a an ein mit der Zuführvorrichtung 30 verbundenes weiteres System, beispielsweise eine Analyseeinheit, zu übergeben.

Zum Transport der Petrischalen 12a aus dem Stapel 12b von Petrischalen 12a zur Übergabeplatte 60 wird ein mit einem Stapel 12b von Petrischalen 12a beladener Aufnahmeständer 34 in den kurvenförmigen Abschnitt 44b der Endlosfördereinheit 38 bewegt. Am dem Entladebereich 46 der Zuführvorrichtung 30 zugeordneten Ende ist eine Liftgabel 50 vorgesehen, die sich im Wartezustand unterhalb des kurvenförmigen Abschnitts 44b der Endlosfördereinheit 38 befindet. Am deutlichsten ist die Liftgabel 50 aus Fig. 1 ersichtlich. Über eine an der liniearen Verbindungsschiene 58 angeordnete Liftschiene 52 ist die Liftgabel 50 vertikal entlang einer Liftachse L verfahrbar. Als Liftantrieb ist ein mit der Steuereinheit 49 verbundener herkömmlicher Elektromotor 54 bereitgestellt, der auf der von der Liftgabel 50 abgewandten Seite der Liftschiene 52 angeordnet ist und über einen Zahnriemen die Bewegung der Liftgabel antreibt.

Ein an der Verbindungsschiene 58 angeordneter fotoelektrischer Sensor 56a ist ebenso mit der Steuereinheit 49 verbunden. Sobald vom fotoelektrischen Sensor 56a und in der Bodenplatte 31 integrierten Gabellichtschranke die Anwesenheit eines mit zumindest einer Petrischale 12a beladenen Aufnahmeständers 34 in den kurvenförmigen Abschnitt 44b der Endlosfördereinheit 38 erfasst wird und ein entsprechendes Signal an die Steuereinheit 49 gesandt wird, steuert die Steuereinheit 49 den Elektromotor 54 zum Verfahren der Liftgabel 50 an. Die Liftgabel 50 verfährt entlang der Liftschiene 52 unter den Stapel 12b von Petrischalen 12a und weiter mit dem Stapel 12b von Petrischalen 12a in Richtung der Übergabeplatte 60.

In die Übergabeplatte 60 ist eine im Wesentlichen kreisförmige Ausnehmung 62 eingebracht, deren Durchmesser mit 9,4 cm ausreichend groß bemessen ist, dass Petrischalen 12a aller gängigen Größen hindurchbewegt werden können. Die Liftgabel 50 verfährt so weit in Richtung der Übergabeplatte 60, bis die oberste Petrischale 12a durch die Ausnehmung 62 hindurch in eine in Fig. 4 dargestellte Übergabeposition ÜP gelangt.

In der Übergabeposition ÜP ist die oberste Petrischale 12a vollständig auf der Oberseite der Übergabeplatte 60 aus der Ausnehmung 62 herausgetreten, so dass sie horizontal auf der Übergabeplatte 60 verschiebbar ist. Das Erreichen der Übergabeposition ÜP wird durch einen auf der Übergabeplatte 60 angeordneten fotoelektrischen Sensor 56b erfasst und an die Steuereinheit 49 gemeldet, worauf die Steuereinheit 49 den Elektromotor 54 ansteuert, so dass die vertikale Bewegung der Liftgabel 50 angehalten wird.

Fig. 4 zeigt die Zuführvorrichtung 30 mit einer in der Übergabeposition ÜP lagernden Petrischale 12a. Auf der Übergabeplatte 60 ist ein Pusher 64 angeordnet, der über zwei parallel zueinander verlaufende Schienen 66 entlang der Übergabeplatte 60 verfahrbar ist. In einer in den Figuren 1 bis 4 gezeigten Ausgangsposition AP befindet sich der Pusher 64 oberhalb der Liftschiene 52, wobei der Bereich oberhalb der Ausnehmung 62 nicht überdeckt wird, so dass eine Petrischale 12a wie zuvor beschrieben in die Übergabeposition ÜP gebracht werden kann. Die in der Übergabeposition ÜP auf der Liftgabel 50 liegende Petrischale 12a wird vom Pusher 64 auf der Übergabeplatte 60 in eine Übergaberichtung ÜR verschoben.

Im mit einem weiteren System, beispielsweise einer Analyseeinheit, verbundenen Zustand des Zuführsystems 10 befindet sich am von der Ausnehmung 62 entfernten Ende der Übergabeplatte 60 eine Beförderungsvorrichtung, die die Petrischalen 12a übernimmt und beispielsweise in eine Kamera-Kammer fördert.

Sobald die Petrischale 12a vom Pusher 64 von der Übergabeposition ÜP weg verschoben wurde und der Pusher 64 wieder die Ausgangsposition AP eingenommen hat, verfährt die Liftgabel 50 weiter entlang der linearen Liftschiene 52 in Richtung der Übergabeplatte 60, bis die nächste Petrischale12a in die Übergabeposition ÜP gelangt.

Nachdem die unterste Petrischale 12a eines Stapels 12b auf der Übergabeplatte 60 verschoben wurde, verfährt die Liftgabel 50 in die zuvor beschriebene Position unterhalb des kurvenförmigen Abschnitts 44b der Endlosfördereinheit 38. Ein weiterer mit einem Stapel 12b von Petrischalen 12a beladener Aufnahmeständer 34 wird in den kurvenförmigen Abschnitt 44b der Endlosfördereinheit 38 bewegt. Nun können die Petrischalen 12a des nächsten Stapels 12b von Petrischalen 12a zum mit der Zuführvorrichtung 30 verbundenen System transportiert werden.

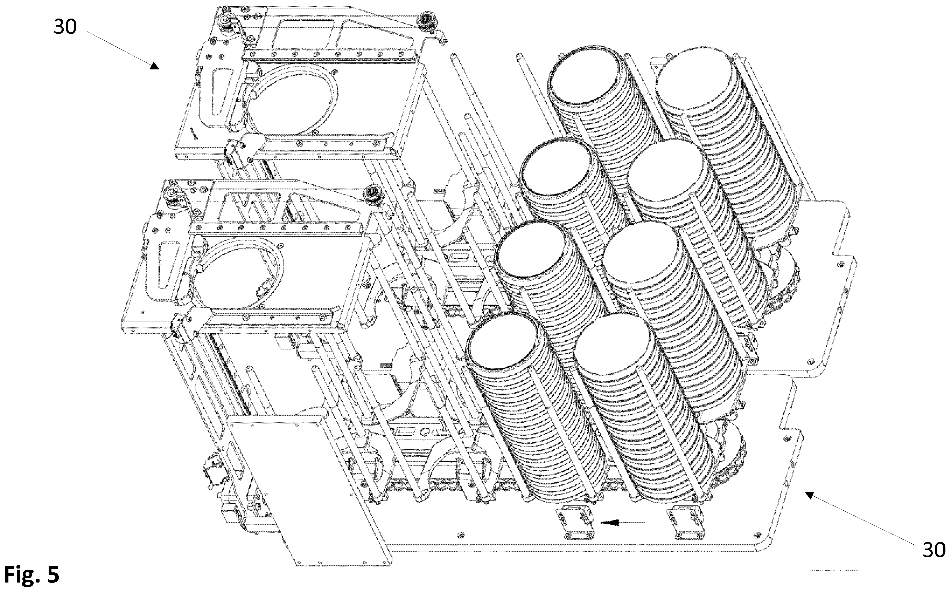

Fig. 5 zeigt eine Ausführungsform der Erfindung mit zwei parallel zueinander angeordneten Zuführvorrichtungen 30. Durch die Anordnung mehrerer Zuführvorrichtungen 30 nebeneinander kann die Aufnahmekapazität des Zuführsystems 10 auf ein Mehrfaches erhöht werden.

### Bezugszeichenliste

- 10: Zuführsystem
- 12: Träger
- 12a: Petrischale
- 12b: Stapel von Petrischalen 12a
- 14: Gehäuse
- 16: Aufnahme
- 18: Oberseite
- 18a: Ladeöffnung
- 20: Unterseite
- 20a: Entladeöffnung
- 22: Verschließmechanismus
- 24: Schieber
- 26: Ausnehmung
- 28: Zapfen
- 28a: Mikroschalter
- 29: Antriebsmotor
- 30: Zuführvorrichtung
- 31: Bodenplatte
- 32: Positionserfassung
- 34: Aufnahmeständer
- 36: Ladebereich
- 38: Endlosfördereinheit
- 40: Förderkette
- 42a, b: geradlinige Abschnitte
- 44a, b: kurvenförmige Abschnitte
- 46: Entladebereich
- 47: Zahnrad
- 48: Antriebsmotor
- 48a: Übersetzungsgetriebe
- 49: Steuereinheit
- 50: Liftgabel
- 52: Liftschiene
- 54: Elektromotor
- 56a, b, c: fotoelektrische Sensoren
- 58: Verbindungsschiene
- 60: Übergabeplatte
- 62: Ausnehmung
- 64: Pusher
- 66: Schienen

- L: Liftachse
- M: Mittelachse
- AP: Ausgangsposition
- ÜP: Übergabeposition
- ÜR: Übergaberichtung

## Patentansprüche

1. Zuführsystem (10) mit einer Zuführvorrichtung (30) zur Förderung von Laborgefäßen für Proben, Mikroorganismen, Zellkulturen oder ähnlichem, und einem Träger (12) mit einer oder mehreren Aufnahmen (16) zum Lagern von Laborgefäßen, wobei die Zuführvorrichtung (30) einen Ladebereich (36) und einen von dem Ladebereich (36) entfernt angeordneten Entladebereich (46) sowie mehrere Aufnahmeeinheiten (34) aufweist, in denen mehrere Laborgefäße übereinander gestapelt lagerbar sind, wobei jede Aufnahmeeinheit (34) mit einer Endlosfördereinheit (38) gekoppelt ist, welche die Aufnahmeeinheit (34) von dem Ladebereich (36) zu dem Entladebereich (46) fördert, und wobei über den Träger (12) im Ladebereich (36) Laborgefäße in eine oder mehrere Aufnahmeeinheiten (34) einbringbar sind, dafür der Träger (12) über die zu beladende oder über die zu entladende zumindest eine Aufnahmeeinheit (34) zumindest bereichsweise gestülpt ist, und dafür dem Träger (12) zugeordnete Vorsprünge (28, 32) und/oder Ausnehmungen im Ladebereich (36) der Zuführvorrichtung (30) aufweist, welche bei in den Ladebereich (36) eingebrachtem Träger (12) zwischen Zuführvorrichtung (30) und Träger (12) einen Formschluss bildet, **dadurch gekennzeichnet, dass** der Träger (12) zumindest zwei Aufnahmen (16) aufweist und dass der Formschluss zwischen Träger (12) und Aufnahmeeinheit (34) im Ladebereich (36) der Zuführvorrichtung (30) nur eine einzige vorbestimmte Ausrichtung des Trägers (12) im Ladebereich (36) ermöglicht.

2. Zuführsystem nach Anspruch 1, **dadurch gekennzeichnet, dass** bedarfsweise, insbesondere bei einer Störung, die Aufnahmeeinheit (34) durch die Endlosfördereinheit (38) in den Ladebereich (36) zurück verfahrbar ausgebildet ist.

3. Zuführsystem nach Anspruch 1 oder 2, **gekennzeichnet durch** eine Formgebung der Aufnahmeeinheit (34), so dass Stapel (12b) von Laborgefäßen (12a) nur in einer definierten Orientierung eingesetzt werden können.

4. Zuführsystem nach Anspruch 3, **dadurch gekennzeichnet, dass** ein Einsetzen der Trägereinheit (12) in einer Orientierung, die nicht der vorgegebenen entspricht, durch eine mechanische Barriere (28, 32) verhindert wird.

5. Zuführsystem nach einem der vorangehenden Ansprüche, **dadurch gekennzeichnet, dass** eine Steuereinheit (49) vorgesehen ist, welche die Lage der Aufnahmeeinheit (34) in dem Ladebereich (36) bei der Beladung erfasst und speichert, so dass bedarfsweise die Aufnahmeeinheit (34) in ihre ursprüngliche Lage im Ladebereich (36) zurückverfahren kann und somit einer vorbestimmten Aufnahme (16) im in den Ladebereich (36) eingebrachten Träger (12) zugeordnet bleibt.

6. Zuführsystem nach einem der vorangehenden Ansprüche, **dadurch gekennzeichnet, dass** ein Sensor(28a), im Ladebereich (36) der Zuführvorrichtung (30) vorgesehen ist, der erfasst, ob ein Träger (12) in dem Ladebereich (36) angeordnet ist, und der vorzugsweise mit der Steuereinheit (49) so zusammenwirkt, dass ein Fördern der Endlosfördereinheit (38) blockiert wird, wenn ein Träger (12) im Ladebereich (36) angeordnet ist.

7. Zuführsystem nach einem der vorangehenden Ansprüche, **dadurch gekennzeichnet, dass** im Ladebereich (36) ein Entriegelungsmechanismus (28, 26) für den Träger (12) vorgesehen ist, der bedarfsweise den in den Ladebereich (36) eingebrachten Träger (12) öffnet, so dass Laborgefäße in eine Aufnahmeeinheit (34) überführt werden können, oder schließt, so dass in einer Aufnahmeeinheit (34) befindliche Laborgefäße wieder entnommen werden können.

8. Zuführsystem nach einem der vorangehenden Ansprüche, **dadurch gekennzeichnet, dass** im Ladebereich (36) Sensoren (54a, 54c) vorhanden sind, welche erfassen, ob in der Aufnahmeeinheit (34) zumindest ein Laborgefäß vorhanden ist, wobei die Sensoren (54a, 54c) vorzugsweise durch optoelektronische Sensoren wie Lichtschranken gebildet werden.

9. Zuführsystem nach einem der vorangehenden Ansprüche, **dadurch gekennzeichnet, dass** die Vorsprünge für den Formschluss des Trägers (12) zugleich auch die in die Aufnahmeeinheit (34) eingebrachten Laborgefäße seitlich begrenzen.

10. Zuführsystem nach einem der vorangehenden Ansprüche, **dadurch gekennzeichnet, dass** einer Aufnahme des Trägers (12) lediglich eine Aufnahmeeinheit (34) zugeordnet ist.

11. Zuführsystem nach Anspruch 8, **dadurch gekennzeichnet, dass** der Träger (12) eine bestimmte Zahl von Aufnahmen (16) für die Laborgefäße aufweist und dass der Ladebereich (36) der Zuführvorrichtung (30) durch eine Zahl Aufnahmeeinheiten (34) gebildet ist, welche der Zahl von Aufnahmen (16) des Trägers (12) entspricht, so dass bei eingebrachtem Träger (12) alle Aufnahmeeinheiten (34) im Ladebereich (36) beladen oder entladen werden können.

12. Zuführsystem nach einem der vorangehenden Ansprüche, **dadurch gekennzeichnet, dass** die Endlosfördereinheit (38) eine Bahn beschreibt, die geradlinige Abschnitte (42a, 42b) und kurvenförmige Abschnitte (44a, 44b) aufweist.

13. Zuführsystem nach einem der vorangehenden Ansprüche, **dadurch gekennzeichnet, dass** zumindest ein ganzer Stapel von Laborgefäßen als Stapeleinheit von oben her mittels des Trägers (12) in eine Aufnahmeeinheit (34) einbringbar ist, insbesondere vier Stapeleinheiten auf einmal in vier Aufnahmeeinheiten (34).

14. Zuführsystem nach einem der vorangehenden Ansprüche, **dadurch gekennzeichnet, dass** jedes Laborgefäß und jeder Stapel gekennzeichnet ist, so dass jedes Laborgefäß und jeder Stapel einer Position im Ladebereich (36) und einer Position im Träger (12) zuordenbar ist.

## Claims

1. Supply device (10) having a supply apparatus (30) for conveying laboratory vessels for samples, microorganisms, cell cultures or the like, and a carrier (12) having one or plural holders (16) for storing laboratory vessels, which supply device (30) has a loading area (36) and an unloading area (46) that is located remote from the loading area (36), as well as a plurality of receiving units (34) in which a plurality of laboratory vessels can be stored in a vertically stacked configuration thereof, with each receiving unit (34) being coupled to an endless conveyor unit (38) which transports the receiving unit (34) from the loading area (36) to the unloading area (46), and in which the carrier (12) can be used to introduce laboratory vessels into one or plural receiving units (34) in the loading area (36), for which purpose the carrier (12) is at least partially slid over the respective at least one receiving unit (34) to be loaded or unloaded, and for this purpose has projections (28, 32) and/or recesses that are associated with the carrier (12) and are provided in the loading area (36) of the supply device (30), which will result in positive locking of the supply device (30) and the carrier (12) when the carrier (12) has been inserted in the loading area (36), **characterized in that** the carrier (12) has at least two holders (16) and that the positive locking of the carrier (12) and the receiving unit (34) in the loading area (36) of the supply device (30) will allow only a single predefined orientation of the carrier (12) in the loading area (36).

2. Supply device according to claim 1, **characterized in that,** if necessary, in particular in the event of a malfunction, the receiving unit (34) is designed such that it can be returned to the loading area (36) by the endless conveyor unit (38),

3. Supply device according to one of claims 1 or 2, **characterized by** a design of the receiving unit (34) that will allow stacks (12b) of laboratory vessels (12a) to be inserted only in a defined orientation thereof.

4. Supply device according to claim 3, **characterized in that** inserting the carrier unit (12) in an orientation which does not correspond to the defined one will be prevented by a mechanical barrier (28, 32).

5. Supply device according to any one of the preceding claims, **characterized in that** a control unit (49) is provided which detects and stores the position of the receiving unit (34) in the loading area (36) during loading so that, if necessary, the receiving unit (34) can be returned to its original position in the loading area (36) and thus remains associated with a predetermined holder (16) in the carrier (12) introduced into the loading area (36).

6. Supply device according to any one of the preceding claims, **characterized in that** a sensor (28a) is provided in the loading area (36) of the supply device (30) which detects whether a carrier (12) is present in the loading area (36) and which preferably cooperates with the control unit (49) so as to prevent a conveying action of the endless conveyor unit (38) when a carrier (12) is present in the loading area (36).

7. Supply device according to any one of the preceding claims, **characterized in that** an unlocking mechanism (28, 26) for the carrier (12) is provided in the loading area (36), which mechanism will act to open the carrier (12) introduced into the loading area (36), if necessary, so that laboratory vessels can be transferred into a receiving unit (34), or to close it so that any laboratory vessels present in a receiving unit (34) can be removed again.

8. Supply device according to any one of the preceding claims, **characterized in that** sensors (54a, 54c) are provided in the loading area (36) which detect whether at least one laboratory vessel is present in the receiving unit (34), which sensors (54a, 54c) preferably take the form of optoelectronic sensors such as light barriers.

9. Supply device according to any one of the preceding claims, **characterized in that** the protrusions for the positive locking of the carrier (12) at the same time also constitute a lateral boundary for the laboratory vessels introduced into the receiving unit (34).

10. Supply device according to any one of the preceding claims, **characterized in that** a single receiving unit (34) only is allocated to a holder of the carrier (12).

11. Supply device according to claim 8, **characterized in that** the carrier (12) has a certain number of holders (16) for the laboratory vessels, and that the loading area (36) of the supply device (30) is constituted by a plurality of receiving units (34) which corresponds to the number of holders (16) of the carrier (12) so that, with the carrier (12) in place, all receiving units (34) in the loading area (36) can be loaded or unloaded.

12. Supply device according to any one of the preceding claims, **characterized in that** the endless conveyor unit (38) runs in a path which has linear segments (42a, 42b) and curved segments (44a, 44b).

13. Supply device according to any one of the preceding claims, **characterized in that** at least one entire stack of laboratory vessels can be introduced as a stacked unit from above into a receiving unit (34) by means of the carrier (12), in particular four stacked units each can be introduced into four receiving units (34) at a time.

14. Supply device according to any one of the preceding claims, **characterized in that** each laboratory vessel and each stack is marked such that each laboratory vessel and each stack can be allocated to a position in the loading area (36) and to a position in the carrier (12).

## Revendications

1. Système d'amenée (10) comprenant un dispositif d'amenée (30) pour le transport de récipients de laboratoire destinés à des échantillons, des micro-organismes, des cultures cellulaires ou similaires, et un support (12) comprenant un ou plusieurs logements (16) pour le stockage de récipients de laboratoire, dans lequel le dispositif d'amenée (30) comprend une zone de chargement (36) et une zone de déchargement (46) agencée à distance de la zone de chargement (36) ainsi que plusieurs unités de logement (34), dans lesquelles plusieurs récipients de laboratoire peuvent être stockés de manière empilée les uns sur les autres, dans lequel chaque unité de logement (34) est accouplée à une unité de transport sans fin (38), laquelle transporte l'unité de logement (34) de la zone de chargement (36) à la zone de déchargement (46), et dans lequel les récipients de laboratoire peuvent être introduits dans une ou plusieurs unités de logement (34) par l'intermédiaire du support (12) dans la zone de chargement (36), à cette fin le support (12) est mis au moins par endroits au-dessus de l'unité de logement (34) à charger ou au-dessus de la au moins une unité de logement (34) à décharger, et à cette fin présente des parties saillantes (28, 32) et/ou des évidements associés au support (12) dans la zone de chargement (36) du dispositif d'amenée (30), lesquels forment une coopération de formes entre le dispositif d'amenée (30) et le support (12) lorsque le support (12) est introduit dans la zone de chargement (36), **caractérisé en ce que** le support (12) comprend au moins deux logements (16) et **en ce que** la coopération de formes entre le support (12) et l'unité de logement (34) dans la zone de chargement (36) du dispositif d'amenée (30) ne permet qu'une seule orientation prédéfinie du support (12) dans la zone de chargement (36).

2. Système d'amenée selon la revendication 1, **caractérisé en ce que,** si besoin est, en particulier lors d'une panne, l'unité de logement (34) est conçue de manière à pouvoir être ramenée dans la zone de chargement (36) par l'unité de transport sans fin (38).

3. Système d'amenée selon la revendication 1 ou 2, **caractérisé par** une mise en forme de l'unité de logement (34), de sorte que les piles (12b) de récipients de laboratoire (12a) ne peuvent être utilisées que dans une orientation définie.

4. Système d'amenée selon la revendication 3, **caractérisé en ce qu'**une utilisation de l'unité de support (12) dans une orientation qui ne correspond pas à celle prédéfinie est empêchée par une barrière mécanique (28, 32).

5. Système d'amenée selon l'une quelconque des revendications précédentes, **caractérisé en ce qu'**une unité de commande (49) est prévue, laquelle détecte et met en mémoire la position de l'unité de logement (34) dans la zone de chargement (36) lors du chargement, de sorte que si besoin est, l'unité de logement (34) peut revenir dans la zone de chargement (36) dans sa position initiale et reste donc associée à un logement (16) prédéfini dans le support (12) introduit dans la zone de chargement (36).

6. Système d'amenée selon l'une quelconque des revendications précédentes, **caractérisé en ce qu'**un capteur (28a) est prévu dans la zone de chargement (36) du dispositif d'amenée (30), qui détecte si un support (12) est agencé dans la zone de chargement (36), et qui coopère de préférence avec l'unité de commande (49) de sorte qu'un transport de l'unité de transport sans fin (38) est bloqué lorsqu'un support (12) est agencé dans la zone de chargement (36).

7. Système d'amenée selon l'une quelconque des revendications précédentes, **caractérisé en ce qu'**un mécanisme de déverrouillage (28, 26) pour le support (12) est prévu dans la zone de chargement (36), lequel si besoin ouvre le support (12) introduit dans la zone de chargement (36), de sorte que les récipients de laboratoire peuvent être transférés dans une unité de logement (34), ou le ferme, de sorte que les récipients de laboratoire situés dans une unité de logement (34) peuvent à nouveau être retirés.

8. Système d'amenée selon l'une quelconque des revendications précédentes, **caractérisé en ce que** des capteurs (54a, 54c) sont présents dans la zone de chargement (36), lesquels détectent si au moins un récipient de laboratoire est présent dans l'unité de logement (34), dans lequel les capteurs (54a, 54c) sont formés de préférence par des capteurs optoélectroniques tels que des barrières lumineuses.

9. Système d'amenée selon l'une quelconque des revendications précédentes, **caractérisé en ce que** les parties saillantes pour la coopération de formes du support (12) délimitent parallèlement également latéralement les récipients de laboratoire introduits dans l'unité de logement (34).

10. Système d'amenée selon l'une quelconque des revendications précédentes, **caractérisé en ce qu'**une seule unité de logement (34) est associée à un logement du support (12).

11. Système d'amenée selon la revendication 8, **caractérisé en ce que** le support (12) comprend un nombre défini de logements (16) pour les récipients de laboratoire et **en ce que** la zone de chargement (36) du dispositif d'amenée (30) est formée par un certain nombre d'unités de logement (34), lequel correspond au nombre de logements (16) du support (12), de sorte que lorsque le support (12) est introduit, toutes les unités de logement (34) peuvent être chargées ou déchargées dans la zone de chargement (36).

12. Système d'amenée selon l'une quelconque des revendications précédentes, **caractérisé en ce que** l'unité de transport sans fin (38) décrit une trajectoire qui présente des parties rectilignes (42a, 42b) et des parties curvilignes (44a, 44b).

13. Système d'amenée selon l'une quelconque des revendications précédentes, **caractérisé en ce qu'**au moins une pile complète de récipients de laboratoire peut être introduite en tant qu'unité empilée par le dessus dans une unité de logement (34) au moyen du support (12), en particulier quatre unités empilées en une fois dans quatre unités de logement (34).

14. Système d'amenée selon l'une quelconque des revendications précédentes, **caractérisé en ce que** chaque récipient de laboratoire et chaque pile sont caractérisés de sorte que chaque récipient de laboratoire et chaque pile peuvent être associés à une position dans la zone de chargement (36) et à une position dans le support (12).
